(19) Europäisches Patentamt

European Patent Office

Office européen des brevets

(11) **EP 0 665 012 B1**

(12) **EUROPEAN PATENT SPECIFICATION**

(45) Date of publication and mention
of the grant of the patent:
**19.09.2001 Bulletin 2001/38**

(51) Int Cl.[7]: **A61K 31/195**, A61K 38/00,
A23L 1/305

(21) Application number: **95101151.9**

(22) Date of filing: **27.01.1995**

(54) **Antiallergy agent and nutritional composition containing glutamine**

Antiallergisches Mittel und Nährstoffzusammensetzung, die Glutamin enthalten

Agent antiallergique et composition nutritive contenant de la glutamine

(84) Designated Contracting States:
**CH DE FR LI NL**

(30) Priority: **31.01.1994 JP 2591394**

(43) Date of publication of application:
**02.08.1995 Bulletin 1995/31**

(73) Proprietor: **SNOW BRAND MILK PRODUCTS CO.,
LTD.
Sapporo-shi, Hokkaido 065 (JP)**

(72) Inventors:
• **Kawakami, Hiroshi
Kawagoe-shi, Saitama (JP)**
• **Yakabe, Takafumi, Pasutel-Rokken-machi A2-2
Kawagoe-shi, Saitama (JP)**
• **Idota, Tadashi, Kawagoe-Green-Park L1-207
Kawagoe-shi, Saitama (JP)**

(74) Representative: **Boeters, Hans Dietrich, Dr. et al
Patentanwälte Boeters & Bauer,
Bereiteranger 15
81541 München (DE)**

(56) References cited:
**EP-A- 0 418 593      EP-A- 0 540 462
WO-A-91/01135      WO-A-92/09277
GB-A- 1 173 576      US-A- 5 039 704**

• **DATABASE BIOSIS BIOSCIENCES
INFORMATION SERVICE, PHILADELPHIA, PA,
US MAZO V K ET AL 'Possible dietary correction
of the macromolecular permeability of the
protective barrier of the gastrointestinal tract' &
VOPROSY PITANIYA, no.5, 1993 pages 10 - 17**
• **PATENT ABSTRACTS OF JAPAN vol. 8, no. 30
(C-209) (1467) 8 February 1984 & JP-A-58 194 815
(NIPPON SHINYAKU K.K.) 12 November 1983**
• **PATENT ABSTRACTS OF JAPAN vol. 8, no. 251
(C-252) (1688) 16 November 1984 & JP-A-59 130
253 (FUJISAWA YAKUHIN KOGYO K.K.) 26 July
1984**
• **PATENT ABSTRACTS OF JAPAN vol. 8, no. 187
(C-240) (1624) 28 August 1984 & JP-A-59 080 644
(FUJISAWA YAKUHIN KOGYO K.K.) 10 May 1984**

**Description**

Field of the Invention

The present invention relates to an antiallergy agent containing glutamine as an active ingredient.

The antiallergy agent in the present invention can be used as an oral pharmaceutical, a nutritional composition or, especially, a nutritional composition for infants and is useful for the prevention of the induction of allergic disease in infancy and the treatment thereof.

Background of the Invention

It is thought that a nutritional composition for infants can not make infants grow as well as infants breast-fed unless they ingest more protein, because it has a different ingredient composition from that of breast milk and its amino acid balance is inferior to that of breast milk.

However, if protein ingestion exceeds the protein requirement, excessive amino acids ingested have to be metabolized, which leads to giving heavy burdens to infants. Consequently, they not only suffer from impairments such as fever, coma, diarrhea, edema, metabolic acidosis and so on, but also have other problems such as elevation of blood-urea level, increase of excretion of phenolic derivatives into urine and abnormal physiological metabolism in infants.

Therefore, for example, the protein content of powder milk or infants has been tried to be lowered as low as that of breast milk. In consequence, there is a report saying that weight gain of infants ingesting such a milk as described above was less than that of infants breast-fed, which suggests that the protein content of powder milk for infants on the present market has reached the lower limit.

Accordingly, it is known that infants breast-fed grow very well in spite of the lower protein content of breast milk than at of a nutritional composition for infants.

From this, it is expected that there is something more than good protein quality of breast milk and the suitable amino acid composition for the healthy growth of human being. We estimated the nitrogen requirement and the energy requirement in infants considering the bioavailability of protein and it is suggested that it is clearly limitative by calculation to maintain healthy growth of infants only by adjusting the protein content of a nutritional composition for infants to the same as that of breast milk.

Protein is a nutrient supplying nitrogen source necessary for a living body and the nitrogen source in breast milk contains on protein nitrogen besides protein.

It was clarified that breast milk usually contained 300 - 8,300 μg /100 ml of glutamine as one of non protein nitrogen ingredients (Japanese Journal of Pediatric Gastroenterology and Nutrition, vol. 5, 209, 1991).

However, even though glutamine is added into a nutritional composition for infants in order for its content to be close to the glutamine content of breast milk, there is still a problem that, due to the unstability of glutamine against heat, added glutamine will change into a nutritional physiologically inactive ingredient cyclised by heating treatment for sterilization in a production process.

Accordingly, it is possible to solve the above nutritional physiological problem in the field of artificial nutrition by the addition of glutamine to nutritional compositions keeping its activity, that is, not only by supplying a nutritional nitrogen source as a non protein nitrogen ingredient but also by the addition of a physiological effect characteristic to glutamine thereto.

In addition, the recent high incidence of allergic disease has lead to a social problem as the number of allergic patients has increased. Especially food allergy which infants and children frequently suffer from became concerned by many people together with atopic dermatitis and infantile asthma.

Though the mechanism of the induction of food allergy symptoms varied, it is principally thought that food allergy is induced by the invasion of allergen having antigenecity (antigen) to living bodies through the undeveloped digestive tract mucosa of infants.

For the prevention or the treatment of food allergy like this, dietary restriction is most generally observed for infants not to take allergen.

However, among food ingredients with allergen like this, there are many high quality of proteins such as egg or milk. And it was clarified that malnutrition affected normal healthy growth when such dietary restriction was carried out during the growing age. And it is also suggested recently that allergic reaction will not be induced if even protein with allergen are skillfully ingested by decreasing its amount.

Additionally from the points of view of preventing allergy and of improving bioavailability, infant formula wherein protein is preliminary digested beforehand is sold on the market.

However, because the unstability of glutamine to heat is not cared at all, there is a problem that a free form of glutamine derived from protein in a production process will lose activity during a heating treatment process.

In a usual amino acid analysis, both of glutamine and glutamic acid are determined as glutamic acid, so that it could not be understood to what extent the glutamine content of a product decreased during a production process. For example, the glutamic acid content (containing glutamine) of powder milk for infants sold on the recent market was determined and the results are as follows:

|  | powder milk for infants (per 100g) mg | infant formula (per 100ml) mg |
|---|---|---|
| A company's | 2818 | 395 |
| B company's | 2344 | 305 |
| C company's | 2300 | 322 |

However, the present inventor separately determined glutamine and glutamic acid contained in protein-hydrolyzed infant formula combined with nutritional compositions, especially with enzymatic hydrolysate of milk protein and surprisingly found that the glutamine content of the products was fairly low.

Additionally it was reported that the concentration of glutamine in the plasma of infants fed infant formula extended with non protein nitrogen ingredients from enzymatically hydrolyzed milk protein was lower than that in the plasma of infants fed normal infant formula by the determination of free form of amino acids (Japanese Journal of Pediatric Gastroenterology and Nutrition, vol. 7, 53, 1993).

Glutamine is thought to be consumed as a nutrient in intestinal epithelium cells and not to be transferred into blood. However, because the difference of the concentration of glutamine in the plasma between the two groups was clarified, it is thought that glutamine will lose its activity or change its property by heating treatment during a production process in the case of nutritional compositions with protein received preliminary enzymatic hydrolysis.

Glutamine is also thought to be an important ingredient of intravenous nutrient because it can rapidly heal wounds after an operation and the nutritional composition and clysis combined with glutamine were disclosed (JP 2119762, JP 3264525).

In addition, techniques using a free form of or a peptide form of glutamine were developed as effective ingredients of intraintestinal nutrient ingested after the operation of intestinal tract for expecting physiological action of repressing degeneration of digestive tract mucosa (JP 5236909).

Further it was disclosed that infant formula were combined with non protein nitrogen ingredients (JP 3240437) but neither the free form of glutamine nor the peptide form of glutamine contained in non protein nitrogen ingredients disclosed therein.

WO-A-92 09277 discloses compositions for oral or parenteral use containing free L-glutamine and also at least one derivative of L-glutamine as a nutrient supply. EP-A-0 540 462 discloses the treatment of a fall in blood L-glutamine levels due to physical activity or overtraining by application of an oral formulation containing L-glutamine or a peptide rich in L-glutamine. GB-A-1 173 576 discloses new polypeptides containing L-glutamine and the therapeutical use of these peptides. US-A-5 039 704 discloses a method for treating catabolic disfunction caused by abnormal glutamine metabolism by application of exogenous glutamine. JP-A-58 194 815 (Patent Abstracts of Japan, vol. 8, no 30 (C-209) (1467), JP-A-59 130 253 (Patent Abstracts of Japan, vol. 8, no. 251 (C-252) (1688) and JP-A-59 080 644 (Patent Abstracts of Japan, vol. 8, no. 187 (C-240) (1624) disclose the use of glutamine derivatives or glutamic acid derivatives for the treatment of allergic diseases or auto-immune diseases.

The nutritional compositions, in the free form of glutamine or the peptide form of glutamine are added, have not been known so far, which means that there has been neither research on the function and efficacy of glutamine for infants other than it being non essential amino acid nor knowledge about these.

From the point of view like this, the present inventors though it very important not only nutritionally but also immunologicall to clarify the physiological function or efficacy of glutamine especially its action for infants, had studied on glutamine found that glutamine had an antiallergy action and made it possible to provide the antiallergy agents containing glutamine as the active ingredient, especially the antiallergy nutritional compositions combined and enriched with glutamine.

The present inventors found that glutamine augmented the protein efficiency ratio in infants and showed an antiallergy effect which resulted in the accomplishment of the present invention.

Summary of the Invention

Accordingly, the present invention provides the use of glutamine in free form or in a peptide form of glutamine or as a mixture of both forms for use of glutamine in free form of glutamine or as a mixture of both forms for manufacturing a medicament for threating and- or preventing allergy.

Other object of the present invention is to provide antiallergy nutritional compositions containing proteins, lipids carbohydrates, vitamins and minerals as main ingredients together with glutamine.

Infant formula in the present specification and claims means milk powder which is prepared for infants.

Powder milk for infants in the present specification and claims means liquid milk which is prepared for infants.

Detailed Description of the Invention and the Referred Embodiments

Glutamine in the present invention can be not only a free form of glutamine, but also a peptide form of glutamine as the main composing amino acid, or a peptide form of glutamine obtained by enzymatic hydrolysis of proteins containing a large amount of glutamine such as gluten.

The antiallergy agents of the present invention can be used in a form of oral pharmaceutical compositions such as tablets, capsules, granules, powders, drinks or in a form of nutritional composition.

As nutritional compositions, especially a form of nutritional composition for infants is preferably used.

Composing ingredients of this composition are proteins, lipids carbohydrates, vitamins and minerals as main ingredients other than glutamine.

The oral pharmaceutical compositions of the present invention can be formed in an appropriate form by combination with pharmaceutical components used usually as pharmaceutical compositions such as extenders, excipients, binders, lubricants and so on or can be used as solutions.

And as nutritional compositions, especially nutritional compositions for infants, infant formula, hydrolyzed infant formula, follow-up milk, specific nutritional infant formula and dried-pulverized powder milk for infants can be exemplified.

The nutritional compositions of the present invention contain proteins, lipids, carbohydrates vitamins and minerals as main ingredients together with glutamine therein.

Glutamine to be added can be a free form of glutamine, a peptide form of glutamine or a mixture of the both forms of glutamine.

As described before, these glutamine forms can be a free form of glutamine sold on the market (for example, L-glutamine of Kanto Kagaku), a peptide form of glutamine sold on the market which are obtained by enzymatic hydrolysis of wheat gluten (for example P [Amano] W-2 of Amano Seiyaku) or, for example, a peptide form of glutamine prepared by the following method:

As disclosed in Japanese laid open publication 5-236909 (1993) gluten of wheat protein or zein of corn protein is hydrolyzed by proteolytic enzymes and peptide fractions are collected after the removal of free amino acids.

Among glutamine forms described above, the peptide form of glutamine is comparative resistant to heating treatment but the free form of glutamine is not resistant to heating and will be inactivated by heating treatment during production process.

Therefore, when the free form of glutamine is used, it is add d to the final products after heating treatment of other r w materials. Especially, when products are dried and pulverize , powder-powder mixing is preferable.

Glutamine is added so that a nutritional composition contains more than 30 mg weight % of glutamine by solid.

The nutritional compositions obtained as described above an augment protein efficiency ratio in infants and prevent allergy. As it is clear from the examination example 3 as described later, the mechanism of action is thought that the permeability of allergenic substances will decrease by the differentiation and maturation of digestive tract mucosa with glutamine,. resulting in making allergic reactions difficult to take place.

It is difficult to obtain the effects of the present invention when the glutamine content is lower than 30 mg weight % in the nutritional compositions by solid.

In addition, the daily glutamine intake is preferably 200 - 2000 mg for adults and 20 - 2000 mg for infants.

As protein, composing the nutritional compositions of the present invention, any protein which is usually used in nutritional compositions, for example, milk protein, hydrolyzed milk thereof treated with enzymes, egg protein, soybean protein and so on an be exemplified.

As carbohydrates, starch, soluble polysaccharide, dextrin, sucrose, lactose, maltose, glucose or artificial sweeteners can be exemplified.

As lipids, oils and fats derived from animals and plants such as, butter, lard, fish oil, palm oil, soybean oil, sunflower oil, rapeseed oil, coconut oil can be exemplified. Any edible oil or fat can be exemplified.

As vitamins, one or more species of any vitamin can be appropriately selected from the group consisting of, for example, vitamin A, B vitamins, vitamin C, vitamin D, vitamin E, K vitamins and so on.

As minerals, calcium, magnesium, potassium, sodium or others an be exemplified.

The nutritional compositions of the present invention can be produced, for example, by mixing or combining glutamines as described above with proteins, carbohydrates, lipids, vitamins minerals and other ingredients in a usual way, followed by he heating treatment for sterilization.

However, in the case of the free form of glutamine, it is preferable that they are mixed or combined after the heating treatment for sterilization.

Additionally these nutritional compositions can be formed in a liquid state or in a pulverized state.

The nutritional compositions obtained in this way show high protein efficiency ratio and an antiallergic effect when infants take them.

As for conventional nutritional compositions, there has been no technical conception of combining glutamine from the point of view of preventing allergy.

In contrast in the present invention the function and the efficacy of glutamine for infants were clarified and the combination of the free form of glutamine or the peptide form of glutamine with nutritional compositions can augment protein efficiency and prevent allergy.

Therefore, when infants take the antiallergy agents of the present invention, especially the antiallergy compositions, the following effects can be exemplified.

1) Healthy growth (weight gain) can be obtained in spite of low protein content thereof.
2) The protein content thereof can be the same as that of breast milk, resulting in lightening the burden of amino acid metabolism of infants.
3) Allergy can be prevented because the invasion of allergens into living bodies can be prevented.

Examination examples to confirm the effects of the nutritional compositions of the present invention containing glutamine are illustrated as follows:

Protein efficiency is evaluated by amino acid score calculated from composing amino acid compositions, chemical score calculated in the same way as above, PER (weight gain method) calculated from animal growth (weight gain), biological value (BV) calculated from nitrogen balance or NPU calculated in the same ways as BV.

Among these parameters, amino acid score and chemical score are calculated by a method to compare amino acid composition of an object protein with that of the whole egg protein or with that of breast milk protein as an ideal amino acid composition. And it is very convenient to evaluate these scores by determining the composing amino acid compositions.

However the evaluation by this method has problems such as follows:

The ideal amino acid composition has extremely provisional properties. And the difference of bioavailability of object proteins is not considered.

Then the present inventors evaluated PER of the nutritional compositions of the present invention by using rats.

Examination example 1

Protein Efficiency Ratio (PER) Examination

(Preparation of the peptide form of glutamine)

200 g of wheat gluten (Nakarai-Tesk) was dissolved in ethanol. The solution was added with stirring to 1 % of acetic acid solution to be suspended.

Gluten in the suspension was treated with molsin (protease type XIII, Sigma) at 37°C for 24 hours and further with actinase (Kaken Seiyaku) at 37°C for 24 hours. During this treatment, more than 90 weight % of glutamine and glutamic acid remained as in peptide having molecular weight of less than 1000 and other amino acids such as valine, phenylalanine, isoleucine and so on became free forms of amino acids. These free amino acids were removed by the treatment with an ultrafiltration membrane whose cut-off molecular weight was 500 and 138 g of the mixed material of tetrapeptide and pentapeptide (gluten peptide) containing more than 40 weight % of glutamine was obtained.

(Preparation of animal diets)

Animal diets for the examination were prepared by combining the following various types of foods with purified soybean oil as a lipid source, α-corn starch as carbohydrate source, cellulose as a food fiber source, mineral mixtures with AIN-76 composition and vitamin mixtures.

1) Standard diet containing 20 weight % of milk casein. (hereinafter referred to standard diet)
2) Low protein diet containing 10 weight % of milk casein. (hereinafter referred to low protein diet)
3) Diet containing 10 weight % of milk casein and 0.008 weight % of the peptide form of glutamine (hereafter in this paragraph referred to glutamine) prepared as described above (hereinafter referred to 0.008 added diet)
4) Diet containing 10 weight % of milk casein and 0.08 weight % of glutamine (hereinafter referred to 0.08 added diet)
5) Diet containing 10 weight % of milk casein and 0.8 weight % of glutamine (hereinafter referred to 0.8 added die )

(PER examination)

As experimental animals, Wistar rats (male, 4 weeks of age; purchased from Charles-River) were used and divided into the following 5 groups so that each group had five rats and the same average body weight after raising preliminarily for 5 days:

1) Standard diet (hereinafter referred to standard diet group)
2) Low protein diet (hereinafter referred to low protein diet group)
3) 0.008 added diet (hereinafter referred to 0.008 added diet group)
4) 0.08 added diet (hereinafter referred to 0.08 added diet group)
5) 0.8 added diet (hereinafter referred to 0.8 added diet group)

They were fed powder diets containing the compositions as described above for 28 days. During the experimental raising, the diet intake and body weight of the experimental animals were daily weighed in principle.

They were raised in stainless cages partitioned for single animal in a room with controlled lighting cycle of each 12 hours, controlled room temperature at $23 \pm 2°C$ and controlled humidity at $55 \pm 10$ %.

During the experiment the diets and water were freely taken.

Comparing the body weight at the beginning of the experiments with that at 28 days after, it was confirmed that weight gain of the 0.08 added diet group and that of the 0.8 added diet group were excellent. The experimental results are shown in Table 1. The fundamental formula for the determination of PER is as follows:

The fundamental formula for the determination of PER

$$PER = \text{weight gain (g)/protein intake (g)}$$

PER is weight gain(g) per 1 g of protein intake and means production efficiency of body composing components by protein ingested.

Unless a significant change in body composition is observed, it can be evaluated as increasing efficiency of body protein. Additionally PER has a characteristic of being able to follow body weight changes for a long duration.

Table 1

| The results of PER examination | | | |
|---|---|---|---|
| | Diet ingested (g) | Body weight gain (g) | PER |
| standard diet group | $402 \pm 26$ | $134.7 \pm 6.4$ | $1.68 \pm 0.65$ |
| low protein diet group | $352 \pm 12$ | $97.4 \pm 3.5$ | $2.76 \pm 0.20$ |
| 0.008 added diet group | $410 \pm 15$ | $110.5 \pm 4.5$ | $2.69 \pm 0.11$ |
| 0.08 added diet group | $396 \pm 10$ | $125.2 \pm 3.0$ | $3.16 \pm 0.09*$ |
| 0.8 added diet group | $430 \pm 9$ | $137.3 \pm 2.9$ | $3.19 \pm 0.05*$ |
| mean values $\pm$ standard deviation (n=5) | | | |

∗ significantly different from the low protein diet group (P<0.05)

Examination example 2

Examination for the confirmation of the elevation of peptidase activity of intestinal tract mucosa.

Small intestines were resected from the rats in the PER examination described above and intestinal brush-border membrane fractions were prepared according to the method of Kawakami and Lonnerdal (Am. J. Physiol. 261, G841, 1991).

By using Lys-Ala-MCA (Peptide Research Foundation) which is a peptide substrate conjugated with 7-amino, 4-methylcumarine (AMC) through amide-bond to carboxyl group of amino acid, activities of dipeptidyl aminopeptidase were determined.

AMC separated by enzymatic reaction was determined by using a fluorometer (excitation wave length: 380 nm, emission wave length: 440 nm).

As a result it was clarified that the enzymatic activity of the 0.08 added diet group and that of the 0.8 added diet group were significantly higher than that of the low protein diet group. The results are shown in Table 2. Determined values are expressed with relative values compared with the enzymatic activity of the intestinal brush-border membrane of the low protein diet group rats as 1.

Table 2

| Enzymatic activities of intestinal brush-border membrane | |
|---|---|
| | dipeptidylaminopeptidase activities |
| low protein diet group | 1 |
| 0.008 added diet group<br>0.08 added diet group<br>0.8 added diet group | $1.2 \pm 0.5$<br>$3.6 \pm 0.9*$<br>$3.9 \pm 1.2*$ |
| mean values $\pm$ standard deviation (n = 5) | |

∗ significantly different from the low protein diet group (P<0.05)

Examination example 3

Examination of inhibitory effect on allergen invasion

Male Wistar rat suckling (14 days of age, n = 10, purchased from Charles-River) were divided into control group consisting of 5 rats and a group administered the peptide form of glutamine consisting of 5 rats.

The sucklings of the control group were raised by breast-feeding as usual. Daily for a week (14 days - 20 days after their birth), the sucklings of the group administered the peptide form of glutamine were orally administered 50 µl of the peptide form of glutamine solution (1 mg/ml) prepared as described before by using micropipets.

Each suckling was orally administered 100 µl of β-lactoglobulin (β-Lg) solution (10 mg/ml) as an antigen on day 21 and the blood samples were taken after one hour and two weeks from the antigen administration.

On the other hand, the β-Lg solution was mixed with Freund's complete adjuvant to form an emulsion and the emulsion was subcutaneously injected into 3 sites of the skin (right dorsal, left dorsal and hip sites) in rabbits with 3 months of age (Japanese white species, male, purchased from Kitayama-rabes), so that anti- β-Lg antiserum was obtained.

The concentration of β-Lg in the blood samples taken after one day from the antigen injection was determined by the sandwich ELISA method by using the antiserum described above as a primary antibody with a secondary antibody labeled with horseradish peroxidase (PO) [The Japanese Journal of Pediatric Allergy nd Clinical Immunology, 1, 36 (1987)].

And the concentration of anti- β-Lg IgE in the blood samples after 2 weeks from the antigen injection was determined by the ELISA (Nordic) method using β-Lg and PO labeled anti-rat IgE antibody. As a result, the group administered the peptide form of glutamine showed lower reactivity to the antigen β-Lg in comparison with the control group. And it was clarified teat glutamine had an antiallergy action. The determination results are shown in Table 3.

Table 3

| The result of the determination of the concentration of β-Lg and anti- β-Lg antibody in the blood. | | |
|---|---|---|
| | β-Lg (ng/ml) | Anti- β-Lg IgE(ng/ml) |
| control group<br>group administered the<br>peptide form of glutamine | $30.6 \pm 15.6$<br>$5.7 \pm 2.9$ | $450.8 \pm 108.6$<br>$126.9 \pm 86.5*$ |
| mean values $\pm$ standard deviation (n=5) | | |

∗ significantly different from the control group (P<0.05)

The present invention is further explained by the following examples but the scope of present invention is not restricted by these examples.

Example 1.

(Preparation of the peptide form of glutamine)

200 g of wheat gluten (Nakarai-Tesk) was dissolved in ethanol.
The solution was added with stirring to 1 % of acetic acid solution to be suspended.

Gluten in the suspension was treated with molsin (protease type XIII, Sigma) at 37°C for 24 hours and further with actinase (Kaken Seiyaku) at 37°C for 24 hours. During this treatment, more than 90 weight % of glutamine and glutamic acid remained as in peptide having molecular weight of less than 1000 and other amino acids such as valine, phenylalanine, isoleucine and so on became free forms of amino acids. These free amino acids were removed by the treatment with an ultrafiltration membrane whose cut-off molecular weight was 500 and 138 g of the mixed material of tetrapeptide and pentapeptide (gluten peptide) containing more than 40 weight % of glutamine was obtained.

(Preparation of powder milk for infants)

100 g of the peptide form of glutamine (glutamine content: 40 g) obtained as described above was dissolved in 700 kg of water with 78 kg of whey powder hydrolyzed enzymatically and 1 kg of vitamins and minerals. In addition, 23.9 kg of plant oil as mixed into the above solution and homogenized, followed by sterilization, condensation and drying, so as to give 100 kg of powder milk for infants.
The protein content of 100 g of the powder milk obtained as above was 13.0 g and the glutamine content thereof was 36 mg.
The glutamine content was determined as the free form of glutamine by an amino acid analyzer (Hitachi, Model 835) after enzymatic hydrolysis of peptide or proteins according to the method of Hill and Schmidt (Journal of Biological Chemistry, 237, 389, 1962).

Example 2.

78 kg of whey powder hydrolyzed enzymatically was dissolved in 700 kg of water with 1 kg of vitamins and minerals.
Further, 23.9 kg of plant oil was mixed with the solution above and homogenized, followed by sterilization, condensation and drying, so as to give 100 kg of powder milk for infants.
This powder milk and 40 g of the free form of glutamine (Kanto Kagaku L-glutamine) were mixed in a powder state.
The glutamine content of the powder milk obtained as above as 40 mg/100 g.

Example 3.

100 g of the peptide form of commercially available glutamine [Amano Seiyaku EP (Amano) W-2] was dissolved in 700 kg of water with 78 kg of whey powder hydrolyzed enzymatically and 1 kg of vitamins and minerals.
Further, 23.9 kg of plant oil was mixed into the above solution and homogenized, followed by sterilization, condensation and drying, so as to give 100 kg of powder milk for infants. The protein content of 100 g of the powder milk obtained as above was 13.0 g and the glutamine content thereof was 33 mg.

Example 4.

The peptide form of glutamine prepared in example 1 was dried, pulverized and filled in soft capsules as an antiallergy agent so that each capsule should contain 500 mg of glutamine.

**Claims**

1.  Use of glutamine in free form or in a peptide form of glutamine or as a mixture of both forms for manufacturing an agent for treating and/or preventing allergies.

2.  The use according to claim 1, wherein said agent is in a form for oral administration.

3.  The use according to claims 1 to 2, wherein said agent is in a form of a nutritional composition.

4.  The use according to claim 3, wherein said nutritional composition contains proteins, lipids, carbohydrates, vitamins and minerals as main ingredients with glutamine.

5.  The use according to claim 4, wherein said nutritional composition is a nutritional composition for infants.

6.  The use according to claim 4 or 5 wherein said nutritional composition contains more than 30 mg weight % of the glutamine in free form or in a peptide form of glutamine or as a mixture of both forms by solid conversion.

7. The use according to any of claims 1 to 6, wherein for said manufacturing combined feedstocks containing proteins, lipids, carbohydrates, vitamins and minerals as main ingredients are combined with the free form of glutamine after heating treatment.

**Patentansprüche**

1. Verwendung von Glutamin in freier Form oder in einer Peptidform von Glutamin oder als Mischung beider Formen zur Herstellung eines Mittels zur Behandlung und/oder Vorbeugung von Allergien.

2. Die Verwendung nach Anspruch 1, wobei das genannte Mittel in einer Form zur oralen Anwendung vorliegt.

3. Die Verwendung nach den Ansprüchen 1 bis 2, wobei das genannte Mittel in Form einer Nahrungsmittelzusammensetzung vorliegt.

4. Die Verwendung nach Anspruch 3, wobei die genannte Nahrungsmittelzusammensetzung Proteine, Lipide, Kohlenhydrate, Vitamine und Mineralien als Hauptbestandteile neben Glutamin enthält.

5. Die Verwendung nach Anspruch 4, wobei die genannte Nahrungsmittelzusammensetzung eine Nahrungsmittelzusammensetzung für Säuglinge ist.

6. Die Verwendung nach Anspruch 4 oder 5, wobei die genannte Nahrungsmittelzusammensetzung mehr als 30 mg Gew.-% Glutamin in freier Form oder in Peptidform von Gluatamin oder einer Mischung beider Formen, nach Überführung in den festen Zustand, enthält.

7. Die Verwendung nach einem der Ansprüche 1 bis 6, wobei für die genannte Herstellung kombinierte Rohprodukte, die als Hauptbestandteile Proteine, Lipide, Kohlenhydrate, Vitamine und Mineralien enthalten, nach einer Hitzebehandlung mit Glutamin in freier Form kombiniert werden.

**Revendications**

1. Utilisation de glutamine sous une forme libre ou sous une forme peptidique de glutamine ou sous la forme d'un mélange des deux formes, pour la fabrication d'un agent pour le traitement et/ou la prévention des allergies.

2. Utilisation selon la revendication 1, dans laquelle ledit agent est sous une forme pour l'administration orale.

3. Utilisation selon la revendication 1 ou 2, dans laquelle ledit agent est sous la forme d'une composition nutritionnelle.

4. Utilisation selon la revendication 3, dans laquelle ladite composition nutritionnelle contient des protéines, des lipides, des hydrates de carbone, des vitamines et des minéraux à titre d'ingrédients principaux avec la glutamine.

5. Utilisation selon la revendication 4, dans laquelle ladite composition nutritionnelle est une composition nutritionnelle pour nourrissons.

6. Utilisation selon la revendication 4 ou 5, dans laquelle ladite composition nutritionnelle contient plus de 30 mg, % en poids, de la glutamine sous une forme libre ou sous une forme peptidique de glutamine ou sous la forme d'un mélange des deux formes, par conversion en solide.

7. Utilisation selon l'une quelconque des revendications 1 à 6, dans laquelle pour ladite fabrication, des réserves alimentaires combinées contenant des protéines, des lipides, des hydrates de carbone, des vitamines et des minéraux à titre d'ingrédients principaux, sont combinées avec la forme libre de glutamine après un traitement thermique.